Europäisches Patentamt

European Patent Office

Office européen des brevets

(11) Publication number : **0 557 076 A1**

# EUROPEAN PATENT APPLICATION

(21) Application number : 93301157.9

(22) Date of filing : 17.02.93

(51) Int. Cl.$^5$ : **C12N 15/17, C12P 21/00**

(30) Priority : **18.02.92 US 837013**

(43) Date of publication of application :
**25.08.93 Bulletin 93/34**

(84) Designated Contracting States :
**AT BE CH DE DK ES FR GB GR IE IT LI LU NL
PT SE**

(71) Applicant : **ELI LILLY AND COMPANY**
**Lilly Corporate Center**
**Indianapolis Indiana 46285 (US)**

(72) Inventor : **Frank, Bruce Hill**
**9377 Spring Forest Drive**
**Indianapolis, Indiana 46260 (US)**
Inventor : **Furman, Thomas Charles**
**4731 Shireton Court**
**Indianapolis, Indiana 46254 (US)**
Inventor : **McDonough, James Patrick**
**3020 Silver Maple Court**
**Carmel, Indiana 46033 (US)**

(74) Representative : **Hudson, Christopher Mark et
al**
**Erl Wood Manor**
**Windlesham Surrey GU20 6PH (GB)**

(54) **Selective removal of N-terminal extensions from folded insulin precursors using
dipeptidyl-aminopeptidase-1.**

(57)    Dipeptide aminopeptidase I selectively removes N-terminal dipeptide extensions from folded insulin
precursors.

EP 0 557 076 A1

Jouve, 18, rue Saint-Denis, 75001 PARIS

The application of recombinant DNA technology to the biosynthesis of proteins in procaryotic cells generally results in expression of a protein having a methionine residue at the amino terminus. Since the addition of methionine to the native protein may alter its biological activity, conformation stability, antigenicity, etc., it is most desirable, if possible, to remove it.

By insertion of a cleavage site between the amino-terminal methionine and the desired native protein, one in theory has a greater degree of flexibility in the methodology selected for achieving production of the desired native protein. In fact, however, there have been only a very limited number of practical methods for achieving selective cleavage. A most important objective thus is to discover alternate methods by which the amino-terminal methionine can be eliminated with generation of the native biosynthetically-produced protein.

One method recognized to be available for the removal of N-terminal amino acids involves the use of cathepsin C, also referred to as dipeptidyl-aminopeptidase-1 (DAP-1). DAP-1 is an enzyme which removes dipeptides (two amino acids, as a unit) from the amino terminus of a protein or polypeptide. Under appropriate conditions, dipeptide removal will commence and continue unless and until (1) the amino group of the N-terminal amino acid is blocked, (2) the site of removal is on either side of a proline, or (3) the N-terminal amino acid is lysine or arginine.

DAP-1 thus may have some usefulness in producing a desired biosynthetically-produced protein from a precursor molecule by designing such precursor molecule to contain an amino acid residue between the initiating methionine and the desired product. Treatment of the precursor molecule with DAP-1 can result in removal of the initiating dipeptide with production of the desired product. This process, however, may be severely limited in its application since dipeptide removal will be expected to continue sequentially until one of the aforedescribed termination sequences is encountered. Thus, the DAP-1 approach should find limited usefulness, being applicable generally only in those instances in which the N-terminal sequence of the desired product contains a DAP-1 stop sequence.

The present invention provides a method for utilizing DAP-1 to selectively remove undesirable N-terminal amino acids from folded insulin precursors (precursors to compounds with insulin-like activity). The present invention allows undesirable N-terminal amino acids such as the methionine residue which is present on the amino terminus of insulin precursors produced in prokaryotes and the leader sequences often used in yeast expression systems, to be selectively removed. Thus, the present invention is a significant advance in the art of the production of compounds having insulin-like activity.

The present invention discloses and claims the surprising discovery that folding of insulin precursors prior to DAP-1 cleavage results in efficient removal of N-terminal dipeptide extensions from the amino terminus of the B-chain of insulin precursors while protecting the insulin precursor from further DAP-1 digestion. McDonald,J.K., Callehan,P.X., and Ellis,S. (Methods in Enzymology 25B, 272-281 (1972)) have shown that the B-chain of oxidized bovine insulin (cysteine residues oxidized to cysteic acid residues, preventing proper folding and disulfide bond formation) which has an N-terminal sequence (residues 1 to 29) identical to human insulin B-chain, is degraded by DAP-1 to dipeptides up to residues 27-30 which are not further degraded due to the emergence of the sequence specific stop at proline 28. Thus, although DAP-1 would be expected to degrade insulin precursors beyond the removal of N-terminal extensions, this invention provides that in the folded state, human proinsulin is not further degraded. Presumably, the folding of the insulin precursor results in a tertiary structure in which the natural amino terminus of the insulin B-chain is protected from DAP-1 cleavage by steric hindrance. Thus, amino terminal extensions of insulin precursors (or any other protein which possessed a similar steric stopping structure) can be removed efficiently without appreciable unwanted digestion of the desired protein. In like fashion, leader sequences, which are genetically engineered into expression system, can also be selectively removed using DAP-1.

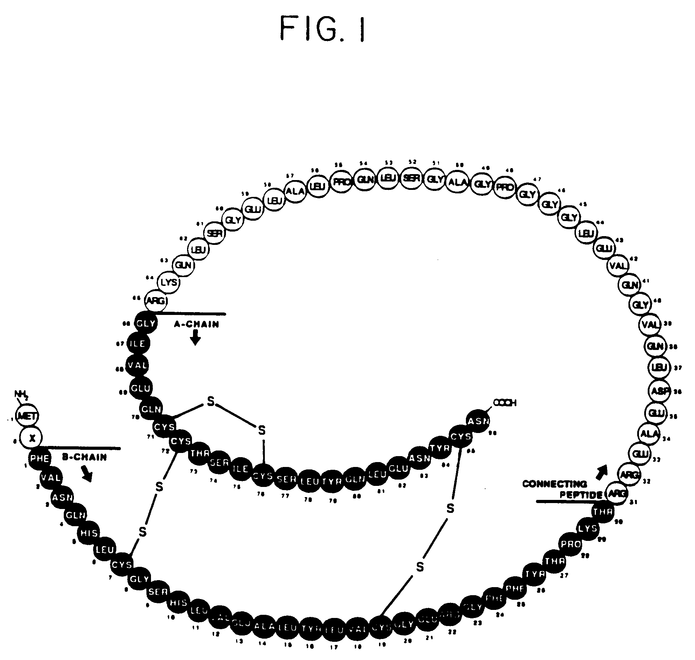

Figure 1 provides the amino acid sequence and disulfide bonding pattern of human proinsulin (residues 1 to 86) with an N-terminal dipeptide extension (residues -1 to 0).

The present invention provides an improved method for removing amino terminal residues from folded insulin precursors with DAP-1. The lack of an environmentally acceptable, yet cost-effective, method for removing N-terminal methionine residues from polypeptide products of interest produced through the genetic engineering of prokaryotes severely limits the applicability of recombinant DNA technology. The present invention provides such a method for removing N-terminal extensions residues from insulin precursors.

The amino acid sequence and disulfide bonding pattern of an illustrative insulin precursor is provided in Figure 1.

As evident from Figure 1, human proinsulin consists of the A-chain (amino acid residues 66 through 86 in Figure 1), the B-chain (amino acid residues 1 through 30 in Figure 1) and connecting peptide (amino acid residues 31 through 65 in Figure 1). Figure 1 also shows an N-terminal dipeptide extension of the B-chain (residues -1 and 0).

The term "insulin precursor" as used herein refers to N-terminal extended species of proinsulin, proinsulin analogs and other proinsulin-like molecules which comprise an insulin A-chain, an insulin B-chain or analogs thereof and which are connected by a connecting peptide, said connecting peptide comprising one or more amino acid residues.

The term "proinsulin analog" comprises proinsulin molecules in which substitutions, deletions and/or additions have been made in the A-chain, and/or B-chain, and/or the connecting peptide.

The term "proinsulin-like molecule" as that term is used in the present invention includes proinsulin and proinsulin analogs. Numerous polypeptides having insulin-like properties are known. A non-exhaustive list of such insulin-like molecules is provided in Table 1. The compounds of Table 1 are listed as either insulin or proinsulin analogs. Skilled artisans realize that an insulin analog differs from a proinsulin analog only by the presence of a connecting peptide or a series of amino acids which are utilized to bridge the A-chain and B-chain.

# Table 1

## Insulin Analogs and Proinsulin Analogs

### A. Single Amino Acid Changes

| | | | | |
|---|---|---|---|---|
| Gly $A_{21}$ | Glu $A_{21}$ | Ser $A_{21}$ | Thr $B_{10}$ | Asp $B_{25}$ |
| Ser $A_{21}$ | Leu $A_{21}$ | Gly $A_{22}$ | Asp $B_{10}$ | His $B_{25}$ |
| Ala $A_{21}$ | Met $A_{21}$ | Ala $A_{22}$ | Arg $B_{10}$ | Glu $B_{26}$ |
| His $A_{21}$ | Tyr $A_{21}$ | Asp $B_9$ | Ile $B_{12}$ | Glu $B_{27}$ |
| Asp $A_{21}$ | Val $A_{21}$ | Asn $B_9$ | His $B_{16}$ | Asp $B_{28}$ |
| Thr $A_{21}$ | Ile $A_{21}$ | His $B_9$ | Gln $B_{17}$ | Ala $B_{30}$ |
| Gln $A_{21}$ | Trp $A_{21}$ | Glu $B_{10}$ | Gln $B_{20}$ | des-$B_{30}$ |
| Thr $A_{30}$-$NH_2$ | Ala $A_{30}$-$NH_2$ | | | |

### B. Two Amino Acid Changes

| | |
|---|---|
| Gly $A_{21}$ and Asp $B_{10}$ | His $A_{21}$ and Lys $B_{27}$ |
| Ser $A_{21}$ and Asp $B_{10}$ | Asp $A_{21}$ and Lys $B_{27}$ |
| Thr $A_{21}$ and Asp $B_{10}$ | Gly $A_{21}$ and Arg $B_{27}$ |
| Ala $A_{21}$ and Asp $B_{10}$ | Ser $A_{21}$ and Arg $B_{27}$ |
| His $A_{21}$ and Asp $B_{10}$ | Thr $A_{21}$ and Arg $B_{27}$ |
| Asp $A_{21}$ and Asp $B_{10}$ | Ala $A_{21}$ and Arg $B_{27}$ |
| Gly $A_{21}$ and Thr $B_{10}$ | Glu $B_{27}$ and Glu $B_{16}$ |
| Ser $A_{21}$ and Thr $B_{10}$ | Asp $B_5$ and Asn $B_{10}$ |
| Thr $A_{21}$ and Thr $B_{10}$ | Glu $B_{12}$ and Gln $B_{13}$ |
| Ala $A_{21}$ and Thr $B_{10}$ | Ser $B_{14}$ and Asp $B_{17}$ |
| His $A_{21}$ and Thr $B_{10}$ | Lys $B_{28}$ and Pro $B_{29}$ |
| Asp $A_{21}$ and Thr $B_{10}$ | His $A_{21}$ and Arg $B_{27}$ |
| Gly $A_{21}$ and Arg $B_{10}$ | Asp $A_{21}$ and Arg $B_{27}$ |

## Table 1 (continued)

Ser $A_{21}$ and Arg $B_{10}$                Glu $B_{12}$ and des-$B_{30}$

Thr $A_{21}$ and Arg $B_{10}$                Asp $B_{10}$ and Ser $B_2$

Ala $A_{21}$ and Arg $B_{10}$                Asp $B_{10}$ and Asp $B_{28}$

His $A_{21}$ and Arg $B_{10}$                Glu $B_{10}$ and Glu $A_{13}$

Asp $A_{21}$ and Arg $B_{10}$                Glu $B_{27}$ and Ser $A_{13}$

Asp $B_{10}$ and des-$B_{30}$                Glu $B_{27}$ and Asp $A_{21}$

Thr $B_{10}$ and des-$B_{30}$                Glu $B_{27}$ and Glu $B_1$

Arg $B_{10}$ and des-$B_{30}$                Glu $B_{27}$ and Asp $B_9$

Gly $A_{21}$ and Lys $B_{27}$                Gly $A_{21}$ and Ala $B_{30}$

Ser $A_{21}$ and Lys $B_{27}$                Ser $A_{21}$ and Ala $B_{30}$

Thr $A_{21}$ and Lys $B_{27}$                Thr $A_{21}$ and Ala $B_{30}$

Ala $A_{21}$ and Lys $B_{27}$                Ala $A_{21}$ and Ala $B_{30}$

des-$B_{29}$ and des-$B_{30}$                hSer $A_{21}$ and Ala $B_{30}$

## C. Three Amino Acid Changes

Gly $A_{21}$ + Lys $B_{27}$ + Gln $A_{17}$

Ser $A_{21}$ + Lys $B_{27}$ + Gln $A_{17}$

Thr $A_{21}$ + Lys $B_{27}$ + Gln $A_{17}$

Ala $A_{21}$ + Lys $B_{27}$ + Gln $A_{17}$

His $A_{21}$ + Lys $B_{27}$ + Gln $A_{17}$

Asp $A_{21}$ + Lys $B_{27}$ + Gln $A_{17}$

Gly $A_{21}$ + Lys $B_{27}$ + Gln $B_{13}$

Ser $A_{21}$ + Lys $B_{27}$ + Gln $B_{13}$

Thr $A_{21}$ + Lys $B_{27}$ + Gln $B_{13}$

Ala $A_{21}$ + Lys $B_{27}$ + Gln $B_{13}$

His $A_{21}$ + Lys $B_{27}$ + Gln $B_{13}$

Asp $A_{21}$ + Lys $B_{27}$ + Gln $B_{13}$

Gly $A_{21}$ + Arg $B_{27}$ + Gln $A_{17}$

## **Table 1(continued)**

Ser $A_{21}$ + Arg $B_{27}$ + Gln $A_{17}$

Thr $A_{21}$ + Arg $B_{27}$ + Gln $A_{17}$

Ala $A_{21}$ + Arg $B_{27}$ + Gln $A_{17}$

His $A_{21}$ + Arg $B_{27}$ + Gln $A_{17}$

Asp $A_{21}$ + Arg $B_{27}$ + Gln $A_{17}$

Gly $A_{21}$ + Arg $B_{27}$ + Gln $B_{13}$

Ser $A_{21}$ + Arg $B_{27}$ + Gln $B_{13}$

Thr $A_{21}$ + Arg $B_{27}$ + Gln $B_{13}$

Ala $A_{21}$ + Arg $B_{27}$ + Gln $B_{13}$

His $A_{21}$ + Arg $B_{27}$ + Gln $B_{13}$

Asp $A_{21}$ + Arg $B_{27}$ + Gln $B_{13}$

Asp $B_{10}$ + His $A_8$ + His $B_{25}$

Glu $B_{10}$ + Glu $A_3$ + Glu $B_{22}$

Glu $B_{27}$ + Ser $B_5$ + Asp $B_5$

Glu $B_{27}$ + His $A_8$ + Asp $B_9$

Glu $B_{27}$ + Asp $A_{21}$ + Asp $B_9$

des-$B_{28}$ + des-$B_{29}$ + des-$B_{30}$

Gly $A_{21}$ + Asp $B_{10}$ + Ala $B_{30}$

Ser $A_{21}$ + Asp $B_{10}$ + Ala $B_{30}$

Thr $A_{21}$ + Asp $B_{10}$ + Ala $B_{30}$

Ala $A_{21}$ + Asp $B_{10}$ + Ala $B_{30}$

His $A_{21}$ + Asp $B_{10}$ + Ala $B_{30}$

Asp $A_{21}$ + Asp $B_{10}$ + Ala $B_{30}$

Gly $A_{21}$ + Thr $B_{10}$ + Ala $B_{30}$

Ser $A_{21}$ + Thr $B_{10}$ + Ala $B_{30}$

Thr $A_{21}$ + Thr $B_{10}$ + Ala $B_{30}$

Ala $A_{21}$ + Thr $B_{10}$ + Ala $B_{30}$

His $A_{21}$ + Thr $B_{10}$ + Ala $B_{30}$

Asp $A_{21}$ + Thr $B_{10}$ + Ala $B_{30}$

Gly $A_{21}$ + Arg $B_{10}$ + Ala $B_{30}$

Ser $A_{21}$ + Arg $B_{10}$ + Ala $B_{30}$

## Table 1(continued)

Thr $A_{21}$ + Arg $B_{10}$ + Ala $B_{30}$

Ala $A_{21}$ + Arg $B_{10}$ + Ala $B_{30}$

His $A_{21}$ + Arg $B_{10}$ + Ala $B_{30}$

Asp $A_{21}$ + Arg $B_{10}$ + Ala $B_{30}$

Gly $A_{21}$ + Asp $B_{10}$ + des-$B_{30}$

Ser $A_{21}$ + Asp $B_{10}$ + des-$B_{30}$

Thr $A_{21}$ + Asp $B_{10}$ + des-$B_{30}$

Ala $A_{21}$ + Asp $B_{10}$ + des-$B_{30}$

His $A_{21}$ + Asp $B_{10}$ + des-$B_{30}$

Asp $A_{21}$ + Asp $B_{10}$ + des-$B_{30}$

Gly $A_{21}$ + Thr $B_{10}$ + des-$B_{30}$

Ser $A_{21}$ + Thr $B_{10}$ + des-$B_{30}$

Thr $A_{21}$ + Thr $B_{10}$ + des-$B_{30}$

Ala $A_{21}$ + Thr $B_{10}$ + des-$B_{30}$

His $A_{21}$ + Thr $B_{10}$ + des-$B_{30}$

Asp $A_{21}$ + Thr $B_{10}$ + des-$B_{30}$

Gly $A_{21}$ + Arg $B_{10}$ + des-$B_{30}$

Ser $A_{21}$ + Arg $B_{10}$ + des-$B_{30}$

Thr $A_{21}$ + Arg $B_{10}$ + des-$B_{30}$

Ala $A_{21}$ + Arg $B_{10}$ + des-$B_{30}$

His $A_{21}$ + Arg $B_{10}$ + des-$B_{30}$

Asp $A_{21}$ + Arg $B_{10}$ + des-$B_{30}$

Thr $B_{10}$ + Glu $B_{28}$ + Pro $B_{29}$

Arg $B_{10}$ + Glu $B_{28}$ + Pro $B_{29}$

Asp $B_{10}$ + Lys $B_{28}$ + Pro $B_{29}$

Thr $B_{10}$ + Lys $B_{28}$ + Pro $B_{29}$

Arg $B_{10}$ + Lys $B_{28}$ + Pro $B_{29}$

Gly $A_{21}$ + Glu $B_{28}$ + Pro $B_{29}$

Ser $A_{21}$ + Glu $B_{28}$ + Pro $B_{29}$

Thr $A_{21}$ + Glu $B_{28}$ + Pro $B_{29}$

Ala $A_{21}$ + Lys $B_{28}$ + Pro $B_{29}$

His $A_{21}$ + Lys $B_{28}$ + Pro $B_{29}$

## Table 1(continued)

Asp A$_{21}$ + Lys B$_{28}$ + Pro B$_{29}$

Glu B$_{28}$ + Pro B$_{29}$ + Ala B$_{30}$

Glu B$_{28}$ + Pro B$_{29}$ + des-B$_{30}$

Lys B$_{28}$ + Pro B$_{29}$ + Ala B$_{30}$

Lys B$_{28}$ + Pro B$_{29}$ + des-B$_{30}$

Arg B$_{27}$ + Gly A$_{21}$ + Thr B$_{30}$-NH2

### D.  Four Amino Acid Changes

Gly A$_{21}$ + Lys B$_{27}$ + Gln A$_{17}$ + Gln B$_{13}$

Ser A$_{21}$ + Lys B$_{27}$ + Gln A$_{17}$ + Gln B$_{13}$

Thr A$_{21}$ + Lys B$_{27}$ + Gln A$_{17}$ + Gln B$_{13}$

Ala A$_{21}$ + Lys B$_{27}$ + Gln A$_{17}$ + Gln B$_{13}$

Asp A$_{21}$ + Lys B$_{27}$ + Gln A$_{17}$ + Gln B$_{13}$

His A$_{21}$ + Lys B$_{27}$ + Gln A$_{17}$ + Gln B$_{13}$

Gly A$_{21}$ + Arg B$_{27}$ + Gln A$_{17}$ + Gln B$_{13}$

Ser A$_{21}$ + Arg B$_{27}$ + Gln A$_{17}$ + Gln B$_{13}$

Thr A$_{21}$ + Arg B$_{27}$ + Gln A$_{17}$ + Gln B$_{13}$

Ala A$_{21}$ + Arg B$_{27}$ + Gln A$_{17}$ + Gln B$_{13}$

Asp A$_{21}$ + Arg B$_{27}$ + Gln A$_{17}$ + Gln B$_{13}$

His A$_{21}$ + Arg B$_{27}$ + Gln A$_{17}$ + Gln B$_{13}$

Glu B$_{10}$ + His A$_{8}$ + His B$_{4}$ + His B$_{27}$

des-B$_{27}$ + des-B$_{28}$ + des-B$_{29}$ + des-B$_{30}$

Gly A$_{21}$ + Asp B$_{10}$ + Glu B$_{28}$ + Pro B$_{29}$

Ser A$_{21}$ + Asp B$_{10}$ + Glu B$_{28}$ + Pro B$_{29}$

Thr A$_{21}$ + Asp B$_{10}$ + Glu B$_{28}$ + Pro B$_{29}$

Ala A$_{21}$ + Asp B$_{10}$ + Glu B$_{28}$ + Pro B$_{29}$

His A$_{21}$ + Asp B$_{10}$ + Glu B$_{28}$ + Pro B$_{29}$

Asp A$_{21}$ + Asp B$_{10}$ + Glu B$_{28}$ + Pro B$_{29}$

Gly A$_{21}$ + Thr B$_{10}$ + Glu B$_{28}$ + Pro B$_{29}$

Ser A$_{21}$ + Thr B$_{10}$ + Glu B$_{28}$ + Pro B$_{29}$

## Table 1(continued)

Thr $A_{21}$ + Thr $B_{10}$ + Glu $B_{28}$ + Pro $B_{29}$

Ala $A_{21}$ + Thr $B_{10}$ + Glu $B_{28}$ + Pro $B_{29}$

His $A_{21}$ + Thr $B_{10}$ + Glu $B_{28}$ + Pro $B_{29}$

Asp $A_{21}$ + Thr $B_{10}$ + Glu $B_{28}$ + Pro $B_{29}$

Gly $A_{21}$ + Arg $B_{10}$ + Glu $B_{28}$ + Pro $B_{29}$

Ser $A_{21}$ + Arg $B_{10}$ + Glu $B_{28}$ + Pro $B_{29}$

Thr $A_{21}$ + Arg $B_{10}$ + Glu $B_{28}$ + Pro $B_{29}$

Ala $A_{21}$ + Arg $B_{10}$ + Glu $B_{28}$ + Pro $B_{29}$

His $A_{21}$ + Arg $B_{10}$ + Glu $B_{28}$ + Pro $B_{29}$

Asp $A_{21}$ + Arg $B_{10}$ + Glu $B_{28}$ + Pro $B_{29}$

Gly $A_{21}$ + Asp $B_{10}$ + Lys $B_{28}$ + Pro $B_{29}$

Ser $A_{21}$ + Asp $B_{10}$ + Lys $B_{28}$ + Pro $B_{29}$

Thr $A_{21}$ + Asp $B_{10}$ + Lys $B_{28}$ + Pro $B_{29}$

Ala $A_{21}$ + Asp $B_{10}$ + Lys $B_{28}$ + Pro $B_{29}$

His $A_{21}$ + Asp $B_{10}$ + Lys $B_{28}$ + Pro $B_{29}$

Asp $A_{21}$ + Asp $B_{10}$ + Lys $B_{28}$ + Pro $B_{29}$

Gly $A_{21}$ + Thr $B_{10}$ + Lys $B_{28}$ + Pro $B_{29}$

Ser $A_{21}$ + Thr $B_{10}$ + Lys $B_{28}$ + Pro $B_{29}$

Thr $A_{21}$ + Thr $B_{10}$ + Lys $B_{28}$ + Pro $B_{29}$

Ala $A_{21}$ + Thr $B_{10}$ + Lys $B_{28}$ + Pro $B_{29}$

His $A_{21}$ + Thr $B_{10}$ + Lys $B_{28}$ + Pro $B_{29}$

Asp $A_{21}$ + Thr $B_{10}$ + Lys $B_{28}$ + Pro $B_{29}$

Gly $A_{21}$ + Arg $B_{10}$ + Lys $B_{28}$ + Pro $B_{29}$

Ser $A_{21}$ + Arg $B_{10}$ + Lys $B_{28}$ + Pro $B_{29}$

Thr $A_{21}$ + Arg $B_{10}$ + Lys $B_{28}$ + Pro $B_{29}$

Ala $A_{21}$ + Arg $B_{10}$ + Lys $B_{28}$ + Pro $B_{29}$

His $A_{21}$ + Arg $B_{10}$ + Lys $B_{28}$ + Pro $B_{29}$

Asp $A_{21}$ + Arg $B_{10}$ + Lys $B_{28}$ + Pro $B_{29}$

Gly $A_{21}$ + Glu $B_{28}$ + Pro $B_{29}$ + Ala $B_{30}$

Ser $A_{21}$ + Glu $B_{28}$ + Pro $B_{29}$ + Ala $B_{30}$

Thr $A_{21}$ + Glu $B_{28}$ + Pro $B_{29}$ + Ala $B_{30}$

Ala $A_{21}$ + Glu $B_{28}$ + Pro $B_{29}$ + Ala $B_{30}$

## Table 1(continued)

His $A_{21}$ + Glu $B_{28}$ + Pro $B_{29}$ + Ala $B_{30}$
Asp $A_{21}$ + Glu $B_{28}$ + Pro $B_{29}$ + Ala $B_{30}$
Gly $A_{21}$ + Lys $B_{28}$ + Pro $B_{29}$ + Ala $B_{30}$
Ser $A_{21}$ + Lys $B_{28}$ + Pro $B_{29}$ + Ala $B_{30}$
Thr $A_{21}$ + Lys $B_{28}$ + Pro $B_{29}$ + Ala $B_{30}$
Ala $A_{21}$ + Lys $B_{28}$ + Pro $B_{29}$ + Ala $B_{30}$
His $A_{21}$ + Lys $B_{28}$ + Pro $B_{29}$ + Ala $B_{30}$
Asp $A_{21}$ + Lys $B_{28}$ + Pro $B_{29}$ + Ala $B_{30}$
Gly $A_{21}$ + Glu $B_{28}$ + Pro $B_{29}$ + des $B_{30}$
Ser $A_{21}$ + Glu $B_{28}$ + Pro $B_{29}$ + des-$B_{30}$
Thr $A_{21}$ + Glu $B_{28}$ + Pro $B_{29}$ + des-$B_{30}$
Ala $A_{21}$ + Glu $B_{28}$ + Pro $B_{29}$ + des-$B_{30}$
His $A_{21}$ + Glu $B_{28}$ + Pro $B_{29}$ + des-$B_{30}$
Asp $A_{21}$ + Glu $B_{28}$ + Pro $B_{29}$ + des-$B_{30}$
Gly $A_{21}$ + Lys $B_{28}$ + Pro $B_{29}$ + des-$B_{30}$
Ser $A_{21}$ + Lys $B_{28}$ + Pro $B_{29}$ + des-$B_{30}$
Thr $A_{21}$ + Lys $B_{28}$ + Pro $B_{29}$ + des-$B_{30}$
Ala $A_{21}$ + Lys $B_{28}$ + Pro $B_{29}$ + des-$B_{30}$
His $A_{21}$ + Lys $B_{28}$ + Pro $B_{29}$ + des-$B_{30}$
Asp $A_{21}$ + Lys $B_{28}$ + Pro $B_{29}$ + des-$B_{30}$
Asp $B_{10}$ + Glu $B_{28}$ + Pro $B_{29}$ + Ala $B_{30}$
Thr $B_{10}$ + Glu $B_{28}$ + Pro $B_{29}$ + Ala $B_{30}$
Arg $B_{10}$ + Glu $B_{28}$ + Pro $B_{29}$ + Ala $B_{30}$
Asp $B_{10}$ + Lys $B_{28}$ + Pro $B_{29}$ + Ala $B_{30}$
Thr $B_{10}$ + Lys $B_{28}$ + Pro $B_{29}$ + Ala $B_{30}$
Arg $B_{10}$ + Lys $B_{28}$ + Pro $B_{29}$ + Ala $B_{30}$
Asp $B_{10}$ + Glu $B_{28}$ + Pro $B_{29}$ + des-$B_{30}$
Thr $B_{10}$ + Glu $B_{28}$ + Pro $B_{29}$ + des-$B_{30}$
Arg $B_{10}$ + Glu $B_{28}$ + Pro $B_{29}$ + des-$B_{30}$
Asp $B_{10}$ + Lys $B_{28}$ + Pro $B_{29}$ + des-$B_{30}$

## Table 1(continued)

$$\text{Thr } B_{10} + \text{Lys } B_{28} + \text{Pro } B_{29} + \text{des-}B_{30}$$
$$\text{Arg } B_{10} + \text{Lys } B_{28} + \text{Pro } B_{29} + \text{des-}B_{30}$$
$$\text{des-}B_{27} + \text{des } B_{28} + \text{des-}B_{29} + \text{des-}B_{30}$$

### E. Five amino acid changes:

$$\text{des-}B_{26} + \text{des-}B_{27} + \text{des-}B_{28} + \text{des-}B_{29} + \text{des-}B_{30}$$

The human insulin-like molecules of Table 1 have biological activity. See Brange, J., et al, Diabetes Care (1990) Vol. 13, No.9, pages 923-954 and U.S. Patent Number 4,916,212, issued April 10, 1990. For purposes of the present invention, insulin precursors are named by designating the amino acid substituted or deleted at the position of the A or B chain where such substitution or deletion occurs. For example, Met-Arg (Lys $B_{28}$, Pro $B_{29}$) proinsulin is an insulin precursor having an N-terminal extension of Met-Arg on a human proinsulin molecule wherein lysine replaces proline at position 28 of the B chain and proline replaces lysine at position 29 of the B chain.

Hormones such as insulin exert biological activity through interactions with receptors specific for a particular hormone or group of hormones. The biological activity of the insulin analogs of Table 1 depends on maintenance of tertiary structures which are capable of interacting with insulin receptors. Thus, the tertiary structure of insulin analogs which retain biological activity is similar.

The method of the present invention exploits the similarity in tertiary structures of folded insulin precursors thereby providing a method of broad applicability in the removal of undesirable amino terminal extensions from insulin precursors. While the present invention is applicable to a vast array of insulin precursor molecules, certain of these insulin precursor molecules are more preferred than others. Insulin molecules of mammalian species possess similar amino acid sequences. As such, the process of the present invention is readily applicable to these insulin precursors of the various mammalian species. For purposes of the present invention, human insulin precursors are preferred.

Highly preferred insulin precursors for use in the present invention include the native human B-chain sequence. Also preferred, however, are those sequences containing, independently, one or more of the following amino acid modifications in the B-chain:

(1) replacement of the Pro in position 28 by any of the following: Asp, Val, Leu, Ile, Arg, His, Lys, Phe, Ala, or Gly. Of these replacements, the following are more preferred: Val, Leu, Ile, Arg, His, or Lys; and, of these, Lys is most preferred.

(2) replacement of the Lys in position 29 by Pro.

(3) replacement of the Phe in position 1 by Asp.

(4) replacement of the Asn in position 3 by Asp.

(5) replacement of the Ser in position 9 by Asp.

(6) replacement of the His in position 10 by Asp.

(7) replacement of the Thr in position 30 by Ala.

(8) deletion of the Thr in position 30.

(9) deletion of B-Chain residues 28 to 30.

(10) deletion of B-Chain residues 27 to 30.

(11) deletion of B-Chain residues 26 to 30.

(12) deletion of B-Chain residue 1.

(13) deletion of B-Chain residues 1 and 2.

Preferred insulin precursors for use in the present invention include the native Human A-chain sequence. Also preferred, however, is that sequence in which the Asn in position 21 of the A chain (position 86 in Figure 1) is replaced by Ala, Asp, Glu, Gln, Gly, Thr, or Ser.

Insulin precursors for use in accordance with the present invention comprise a connecting peptide which joins the A-chain and B-chain of the insulin precursor. A connecting peptide is an essential part of the insulin precursors amenable to the practice of the present invention because otherwise the amino terminal of the A-chain would be available for digestion with DAP-1. The connecting peptide can be any of a wide range of polypeptide structures. The only requirement imposed by the present invention upon the selection of a connecting peptide is that the connecting peptide must allow proper disulfide pairing between the A chain and the B chain during the folding step, which in the present invention precedes the DAP-1 mediated removal of the N-terminal dipeptide extensions. The connecting peptide generally has at least 2, and preferably from about 2 to about 35 and, more preferably, from about 6 to about 35, amino acid residues. Most preferably, the connecting peptide is the "natural" connecting peptide of human proinsulin. The amino acid sequence of the "natural" connecting peptide of human proinsulin is provided in Figure 1 (residues 31 to 65).

Although it is preferred that the connecting peptide be the natural connecting sequence, as indicated above, it can be any of a wide range of shorter peptide sequences. A typical dipeptide useful as a connecting peptide is -Arg-Arg-. In addition, the connecting peptide can be an extension of the foregoing dipeptide, namely those sequences having the formula -Arg-X'-Arg- in which X' represents at least one amino acid residue. Highly preferred connecting peptides are -Arg-Arg-Lys-Arg- as well as longer chain peptides having the structure -Arg-Arg-X²-Lys-Arg- in which X² is at least one amino acid residue and preferably as least two amino acid residues. These latter include the natural connecting peptide.

The N-terminal extensions which can be selectively removed from insulin precursors through the practice of the present invention are numerous. For purposes of the present invention, an N-terminal extension is any sequence of amino acids which precede residue one of the B-chain. Thus, in relation to the structure of human proinsulin (Figure 1) any residue or residues preceding Phe-1 constitutes an N-terminal extension. A principal object of the present invention is to provide a means whereby N-terminal methionine residues can be removed from insulin precursors of genetically engineered prokaryotic origin.

A further objective of the present invention is to provide a means whereby undesirable N-terminal amino acids of insulin precursors other than those produced by prokaryotes can be removed. The advanced state of the art of molecular biology allows skilled artisans to construct recombinant DNA expression vectors to achieve expression of numerous polypeptide products of interest in host cells other than prokaryotes. The present invention can be applied to the removal of N-terminal extensions of insulin precursors regardless of the means used to produce them. In addition, the present invention could be applied to any recombinantly expressed protein or peptide rich possesses an N-terminus with the appropriate steric properties.

The selectivity of N-terminal dipeptide removal from folded insulin precursors is presumably due to steric hindrance rich limits the accessibility of amino terminus of the B-chain of an insulin precursor molecule to DAP-1. Thus, the N-terminal extensions which can be selectively removed from folded insulin precursors without substantial degradation of the desired B-chain or analog thereof is not limited to any particular amino acid sequence except those amino acid sequences rich comprise a natural stop for DAP-1 must be avoided. N-terminal extensions of from 2 to about 8 amino acids are conveniently removed by the method of the present invention. N-terminal extensions rich are preferred contain an even number of amino acids thereby allowing complete removal of the N-terminal extension to yield a desired insulin precursor or analog thereof. Especially preferred N-terminal extensions are Met-Tyr, Met-Arg, and Met-Phe.

The individual steps which comprise the best mode of practicing the present invention are provided in the examples. The preferred means for generating the N-terminal extended human precursors on which the present invention operates is genetic engineering of prokaryotes such as E. coli. Skilled artisans realize that numerous other prokaryotic strains would also function as host cells.

The scientific and patent literature regarding molecular biology/genetic engineering provides skilled artisans with numerous alternative approaches to the construction of insulin precursor expression vectors. The method of the present invention is applicable to any such folded N-terminal extended insulin precursor.

Dipeptidyl-aminopeptidase 1 (DAP-1) is commercially available from Sigma Chemical Company, St. Louis, Missouri 63178-9916.

DAP-1 is activated by the presence of chloride ion (Cl⁻). Thus, in the conversion of the compounds of this invention, Cl⁻ is added to the medium. The amount of chloride ion can be minimal. Indeed, residual amounts present as the result of earlier treatment of the protein in the presence, for example, of sodium chloride are entirely adequate. Thus, the presence of at least a trace amount of chloride ion is sufficient.

Likewise, since the process for converting the compounds of the present invention is enzymatic, a minor amount, relative to the insulin precursor starting material, of DAP-1 is employed. In general, on a molar basis and relative to the protein substrate, the DAP-1 is present in a ratio of from about 1:10,000 to about . 1:200,000.

The reaction generally is conducted in an aqueous medium suitably buffered to obtain and maintain a pH of from about 3.0 to about 8.5. Preferably, the pH of the medium is slightly acidic to neutral, ranging from about 6 to about 7.5.

Conversion of the compounds of this invention is enhanced by the presence of a reagent affording a sulfhydryl moiety. Typical such reagents are β-mercaptoethanol, dithiothreitol, dithioerythritol, β-mercaptoethylamine, cysteine, cysteamine, and the like. Sulfhydryl reagents preferred for use are β-mercaptoethanol, cysteamine, and cysteine, and, most preferably, cysteine.

The sulfhydryl reagent generally is present in the final reaction medium at a molar concentration of from about 0.01 m$\underline{M}$ to about 200 m$\underline{M}$, preferably from about 0.1 m$\underline{M}$ to about 20 m$\underline{M}$.

The reaction is carried out generally at a temperature of from about 10°C to about 45°C for a period of from about 1 hour to about 24 hours.

The examples which follow are provided to further illustrate the present invention and are not limiting on the scope thereof.

## Example 1. DAP-1 Removal of Met-Tyr dipeptide from Folded Met-Tyr-proinsulin

Met-Tyr-human proinsulin (human proinsulin with a Met-Tyr N-terminal extension) was expressed in $\underline{E}$. $\underline{coli}$ as insoluble granules or inclusion bodies. The Met-Tyr-proinsulin granules were solubilized in 7M urea and purified by ion exchange chromatography. After sulfitolysis and solvent exchange, the Met-Tyr-proinsulin was folded in order to form the native disulfide bond pairs and native tertiary structure. U.S. Patent No. 4,430,266 teaches folding of proinsulin-like molecules. The teachings of U.S. Patent No. 4,430,266 are incorporated herein by reference. The folded Met-Tyr-proinsulin was then purified by reversed-phase chromatography. Met-Tyr-proinsulin fractions were pooled and the organic solvent was removed by evaporation. Prior to addition of DAP-1, the pH of purified Met-Tyr-proinsulin (evaporated column eluate) was adjusted to pH 6.8 with NaOH. DAP-1 was added to achieve a concentration of 1 Unit of DAP-1 per gram of Met-Tyr-proinsulin. The DAP-1 cleavage reaction proceeded at 37°C for 6-20 hours. DAP-1 removal of the Met-Tyr dipeptide from Met-Tyr-proinsulin was terminated by acidification of the reaction with HCl to pH 2.5. HPLC analysis of the cleavage reaction confirmed that 80-100% of the Met-Tyr-proinsulin had been converted to the desired product, proinsulin. The conversion rate range reflects the 6 hour digestion (80%) and the 20 hour digestion (100%).

## Example 2. DAP-1 Removal of Met-Arg dipeptide from Folded Met-Arg-proinsulin

Met-Arg-human proinsulin (human proinsulin with a Met-Arg N-terminal extension) was expressed in $\underline{E}$. $\underline{coli}$ as insoluble granules or inclusion bodies. The Met-Arg-proinsulin granules were solubilized in 7M urea and purified by ion exchange chromatography. After sulfitolysis and solvent exchange, the Met-Arg-proinsulin was folded in order to form the native disulfide bond pairs and native tertiary structure. Prior to addition of DAP-1, the pH of purified Met-Arg-proinsulin was adjusted to pH 7.0 with HCl. DAP-1 was added to achieve a concentration of 3 units of DAP-1 per gram of Met-Arg-proinsulin. The DAP-1 cleavage reaction proceeded at 15°C for 12 hours. DAP-1 removal of the Met-Arg dipeptide from Met-Arg-proinsulin was terminated by acidification of the reaction to pH 3.5 with HCl. HPLC analysis of the cleavage reaction confirmed that 95% of the Met-Arg-proinsulin had been removed to generate the desired product, proinsulin.

## Example 3. DAP-1 Removal of Met-Arg Dipeptide from folded Met-Arg (Lys-B-28, Pro-B-29) Proinsulin

The Met-Arg dipeptide of Met-Arg (Lys-B-28, Pro-B-29) human proinsulin was selectively removed in substantial accordance with the teachings of Examples 1 and 2. The results of the DAP-1 digestion were comparable in terms of the selective removal of only the N-terminal Met-Arg dipeptide with those achieved in Examples 1 and 2.

## Example 4. DAP-1 Removal of Met-Tyr dipeptide from folded Met-Tyr (Lys-B-28, Pro-B-29) Proinsulin

The Met-Tyr dipeptide of Met-Tyr (Lys-B-28, Pro-B-29) human proinsulin was selectively removed in substantial accordance with the teachings of Examples 1 and 2. The results of the DAP-1 digestion were comparable in terms of the selective removal of only the N terminal Met-Tyr dipeptide with those achieved in Examples 1 and 2.

## Claims

1. A method for removing N-terminal extensions from folded insulin precursors, said method comprising treatment of the folded insulin precursor with an effective amount of dipeptidyl-aminopeptidase I.

2. The method of Claim 1 wherein said N-terminal extension comprises an N-terminal methionine.

3.   The method of Claim 2 wherein an odd number of amino residues are positioned between the N-terminal methionine and a proinsulin-like molecule.

4.   The method of Claim 3 wherein the odd number of amino acid residues which are positioned between the N-terminal methionine and the proinsulin-like molecule is in the range of from 1 to 7.

5.   The method of Claim 4 wherein 1 amino acid residue is positioned between the N-terminal methionine and the proinsulin-like molecule.

6.   The method of Claim 5 wherein said amino acid residue positioned between the N-terminal methionine and the proinsulin-like molecule is Arg.

7.   The method of Claim 5 wherein said amino acid residue positioned between the N-terminal methionine and the proinsulin-like molecule is Tyr.

8.   The method of Claim 5 wherein said amino acid residue positioned between the N-terminal methionine and the proinsulin-like molecule is Phe.

9.   The method of Claim 6, 7 or 8 wherein said proinsulin-like molecule is human proinsulin.

10.   The method of Claim 6, 7 or 8 wherein said proinsulin-like molecule is $LysB_{28}ProB_{29}$ proinsulin.

# FIG. I

European Patent
Office

**EUROPEAN SEARCH REPORT**

Application Number

| | DOCUMENTS CONSIDERED TO BE RELEVANT | | EP 93301157.9 |
|---|---|---|---|

| Category | Citation of document with indication, where appropriate, of relevant passages | Relevant to claim | CLASSIFICATION OF THE APPLICATION (Int. Cl.5) |
|---|---|---|---|
| Y | EP - A - 0 397 420 (ELI LILLY AND COMPANY) * Abstract; page 2 * -- | 1 | C 12 N 15/17 C 12 P 21/00 |
| D,Y | METHODS IN ENZYMOLOGY, vol. 25, part B, 1972 (New York) J.K. McDONALD et al. "Preparation and Specificity of Dipeptidyl Aminopeptidase I" pages 272-281 * Page 272 * ---- | 1 | |
| | | | **TECHNICAL FIELDS SEARCHED (Int. Cl.5)** |
| | | | C 12 N C 12 P |

The present search report has been drawn up for all claims

| Place of search VIENNA | Date of completion of the search 26-05-1993 | Examiner WOLF |
|---|---|---|

EPO FORM 1503 03.82 (P0401)